# EUROPEAN PATENT APPLICATION

(11) **EP 0 938 873 A2**
(43) Date of publication of application: **01.09.1999**
(21) Application number: 99200455.6
(22) Date of filing: 18.02.1999
(51) Int. Cl.: A61B 19/00

(54) **Positioner for radiation treatment**

(30) Priority: 19.02.1998 US 26067
(71) Applicant: Isravest Ltd., Ramat Aviv Gimel (IL)
(72) Inventor: Moshe, Cin-Gal, Ramat Hasharon 47203 (IL)
(74) Representative: Ungria Lopez, Javier

(57) **Abstract**

A positioner useful in stereotactic radiation treatment of a patient by radiation from a radiation source from which a radiation beam is emitted along a beam axis, the positioner including a source adapter arm arranged to be attached to the radiation source such that the position of the source adapter arm is determined by the position of the radiation source, and a frame adapter arm pivotably attached to the source adapter arm about a pivot axis, the frame adapter arm defining a frame axis intersecting the pivot axis, the pivot axis and the frame axis being selected such that when the source adapter arm is attached to the radiation source, the pivot axis and the frame axis both intersect the beam axis.

## Description

### FIELD OF THE INVENTION

The present invention relates generally to positioning systems for stereotactic radiation treatment, and particularly to a positioner for use with a radiation source housing and patient support table, the positioner ensuring that a radiation beam passes through an isocenter regardless of the relative movement or sagging of the table and the source housing.

### BACKGROUND OF THE INVENTION

It is imperative in stereotactic radiation treatment to ensure that a radiation beam, such as a linear accelerator (LINAC) central beam of radiation, passes as close as possible to the ideal isocenter, i.e., the theoretical intersection of a rotational axis (azimuth axis) of a table which carries the target (patient) and a rotational axis (elevation or roll axis) of the LINAC gantry. In general, the target lies in the patient's head and a stereotactic frame is provided for holding the head fixed with respect to the floor or the treatment table. The purpose of fixing the stereotactic frame is to ensure that the radiation beam always passes substantially through the isocenter during radiation treatment. One way of fixing the stereotactic frame is by mounting it on the table. Another way is to fix the stereotactic frame with respect to the floor. In each case, means are provided for attempting to position the frame such that the target is substantially in the isocenter.

In practice, however, while the heavy gantry moves to various gantry angles, the LINAC head sags. Moreover, the rotary bearing of the LINAC and/or the table are not ideal and this affects concentricity, *inler alia.* Consequently, the central radiation beam does not intersect the ideal isocenter. Thus, in general, unpredictable deformations of the structures supporting the radiation source or the target, as well as errors in determining the positions of the source or the target lead to inaccuracies in delivering the radiation beam to the isocenter.

### SUMMARY OF THE INVENTION

The present invention seeks to provide an improved positioner for use with a radiation source housing and patient support table. The positioner is attachable to a radiation system having two axes of rotation for stereotactic treatment. The two axes may be the turning axes of the LINAC and table, for example. The positioner has its own axes of rotation *independent* of the rotational axes of the radiation system. When the positioner is attached to the radiation system, the rotational axes of the positioner are placed close to, but not necessarily collinear with, the rotational axes of the system. The target is rigidly referenced to the *positioner.* Thus, the target is irradiated with a precision determined by the *positioner* and not the radiation system.

The positioner comprises two rigid arms, one for attachment to the source housing and the other attachable to a stereotactic frame. The two arms pivot about a common axis and define an isocenter that always remains aligned with a radiation bcam emanating from the radiation source regardless of the relative movement or sagging of the table and the radiation source housing. The two arms thus move in precise relation with the azimuthal, elevational and/or roll movements of the gantry and/or table.

There is thus provided in accordance with a preferred embodiment of the present invention a positioner useful in stereotactic radiation treatment of a patient by radiation from a radiation source from which a radiation beam is emitted along a beam axis, the positioner including a source adapter arm arranged to be attached to the radiation source such that the position of the source adapter arm is determined by the position of the radiation source, and a frame adapter arm pivotably attached to the source adapter arm about a pivot axis, the frame adapter arm defining a frame axis intersecting the pivot axis, the pivot axis and the frame axis being selected such that when the source adapter arm is attached to the radiation source, the pivot axis and the frame axis both intersect the beam axis.

In accordance with a preferred embodiment of the present invention the pivot axis and the frame axis are selected such that when the source adapter arm is attached to the radiation source, the pivot axis and the frame axis both intersect the beam axis generally at the same location, and most preferably, substantially at the same location. Preferably the pivot axis and the frame axis are generally orthogonal.

Further in accordance with a preferred embodiment of the present invention the positioner also includes a source adapter arm positioning assembly which adjusts attachment of the source adapter arm to the radiation source.

Still further in accordance with a preferred embodiment of the present invention the positioner also includes a patient support frame pivotably mounted to the frame adapter arm.

Additionally in accordance with a preferred embodiment of the present invention the positioner also includes a frame adapter arm positioning assembly which adjusts mounting of the patient support frame to the frame adapter arm.

In accordance with a preferred embodiment of the present invention the positioner also includes a beam collimator mounted onto the source adapter arm for collimating the radiation beam.

There is also provided in accordance with a preferred embodiment of the present invention a stereotactic radiation treatment system for treatment of a patient by radiation, including a radiation source from which a radiation beam is emitted along a beam axis, and a positioner including a source adapter arm arranged to be attached to the radiation source such that the position of the source adapter arm is determined by the position of the radiation source, and a frame adapter arm pivotably attached to the source adapter arm about a pivot axis, the frame adapter arm defining a frame axis intersecting the pivot axis, the pivot axis and the frame axis being selected such that when the source adapter arm is attached to the radiation source, the pivot axis and the frame axis both intersect the beam axis.

Preferably the stereotactic radiation treatment system also includes a patient support frame with respect to which a patient can be rigidly mounted such that a target area of the patient is located at an intersection of the pivot axis, the frame axis and the beam axis.

In accordance with a preferred embodiment of the present invention the source adapter arm is suspended from the radiation source. Alternatively in accordance with another preferred embodiment of the present invention the source adapter arm is at least partially suspended from the radiation source.

Further in accordance with a preferred embodiment of the present invention the stereotactic radiation treatment system includes a support element for supporting at least one of the source adapter arm and the frame adapter arm.

### BRIEF DESCRIPTION OF THE DRAWINGS

The present invention will be understood and appreciated more fully from the following detailed description, taken in conjunction with the drawings in which:
Fig. 1 is a simplified pictorial illustration of a positioner for stereotactic radiation treatment constructed and operative in accordance with a preferred embodiment of the present invention; and
Fig. 2 is a simplified pictorial illustration of a positioner for stereotactic radiation treatment constructed and operative in accordance with another preferred embodiment of the present invention.

### DETAILED DESCRIPTION OF A PREFERRED EMBODIMENT

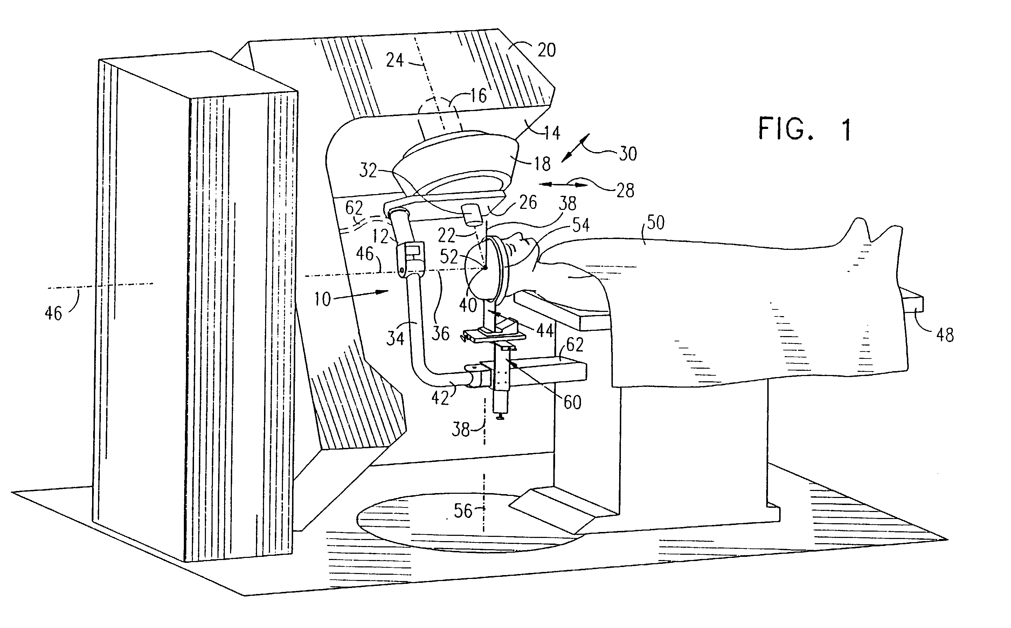

Reference is now made to Fig. 1 which illustrates a positioner 10 for stereotactic radiation treatment, constructed and operative in accordance with a preferred embodiment of the present invention.

Positioner 10 includes a source adapter arm 12 arranged to be attached to a housing 14 of a radiation source 16. For example, arm 12 may be suspended or at least partially suspended from a portion of a tray or turret 18 of a LINAC gantry 20. Radiation source 16 emits a radiation beam 22 along a beam axis 24. Preferably a source adapter arm positioning assembly 26 is provided for adjusting attachment of arm 12 to the radiation source 16. Assembly 26 may adjust the position of arm 12 relative to radiation source 16 along and/or about any of three mutually orthogonal axes, such as axes 28 and 30 (in a plane perpendicular to beam axis 24) and beam axis 24, for example Assembly 26 may include any conventional step motor, actuator or the like, for adjusting the position of arm 12. A beam collimator 32 is preferably mounted onto arm 12 for collimating radiation beam 22. By virtue of the attachment of arm 12 to radiation source 16, the spatial position of arm 12 is determined by the position of radiation source 16.

A frame adapter arm 34 is pivotably attached to source adapter arm 12 about a pivot axis 36. Arm 34 defines a frame axis 38 intersecting pivot axis 36. Pivot axis 36 and frame axis 38 are selected such that when arm 12 is attached to radiation source 16, pivot axis 36 and frame axis 38 both intersect beam axis 24. Preferably pivot axis 36 and frame axis 38 both intersect beam axis 24 generally at the same location, and most preferably substantially at the same location, designated by reference numeral 40. Pivot axis 36 and frame axis 38 are preferably generally orthogonal.

Arm 34 includes a distal portion 42 which is preferably attached to a patient support frame 44 such that frame 44 is rotatable about frame axis 38. As seen in Fig. 1, frame axis 38 is preferably the azimuth axis and pivot axis 36 is preferably the roll axis.

Gantry 20, comprising therein radiation source 16, is rotatable about a gantry axis 46, as is well known in the art. Radiation source 16 generally rotates about gantry axis 46 synchronously with rotation of either one or both of arms 12 and 34 about pivot axis 36. Gantry axis 46 ideally generally coincides with pivot axis 36 However, it is a particular feature of the present invention that even if gantry axis 46 is misaligned with pivot axis 36, source adapter arm 12 always positions location 40 (located at the isocenter) in a desired orientation with respect to radiation source 16, i.e., radiation beam 22 substantially intersects location 40 no matter how gantry 20 has moved or sagged. Source adapter arm 12 succeeds in accurately positioning location 40 relative to radiation source 16 by virtue of arm 12 being stiff and rigidly suspended, or at least partially suspended, from housing 14.

Alternatively, in accordance with another preferred embodiment of the present invention, arms 12 and 34 can be configured or positioned such that pivot axis 36 is *purposely* misaligned with (i.e., offset from) gantry axis 46 so as to enable carrying out an irradiation schemes which may not be possible with rotation about gantry axis 46 of LINAC gantry 20.

There is preferably provided a table 48 upon which a patient 50 may be supported. Patient 50 has a target 52 which is to be irradiated by radiation beam 22. In the illustrated example, the head of patient 50 is affixed to a head ring 54 of frame 44 such that location 40 lies in target 52. Table 48 is rotatable about a table axis 56, as is well known in the art. Table 48 generally, rotates about table axis 56 synchronously with rotation of either one or both of arms 12 and 34 about frame axis 38. Table axis 56 ideally generally coincides with frame axis 38 In a similar fashion as stated hereinabove, it is a particular feature of the present invention that even if table axis 56 is misaligned with frame axis 38, source adapter arm 12 always positions isocenter 40 in a desired orientation with respect to radiation source 16 and frame adapter arm 34 always positions isocenter 40 in a desired orientation with respect to patient support frame 44, i.e., radiation beam 22 substantially intersects location 40 and target 52 no matter how gantry 20 or table 48 has moved or sagged. Radiation source 16 thus "drags" the target of the patient with it no matter where it goes or how it sags, and accurate irradiation of the target is ensured.

A frame adapter arm positioning assembly 60, such as an X-Y-Z positioner, is preferably pivotably mounted to frame adapter arm 34 and attached to patient support frame 44. Assembly 60 adjusts mounting of patient support frame 44 to arm 34. Assembly 60 moves patient support frame 44 along three mutually orthogonal axes (typically corresponding to axes 36 and 38 and to beam axis 24), so that target 52 can be properly aligned with location 40.

Although arms 12 and 34 are preferably stiffly cantilevered from turret 18, if desired, a support element 62 may be optionally provided for supporting either one of arms 12 or 34 without hindering the free movement of the arms.

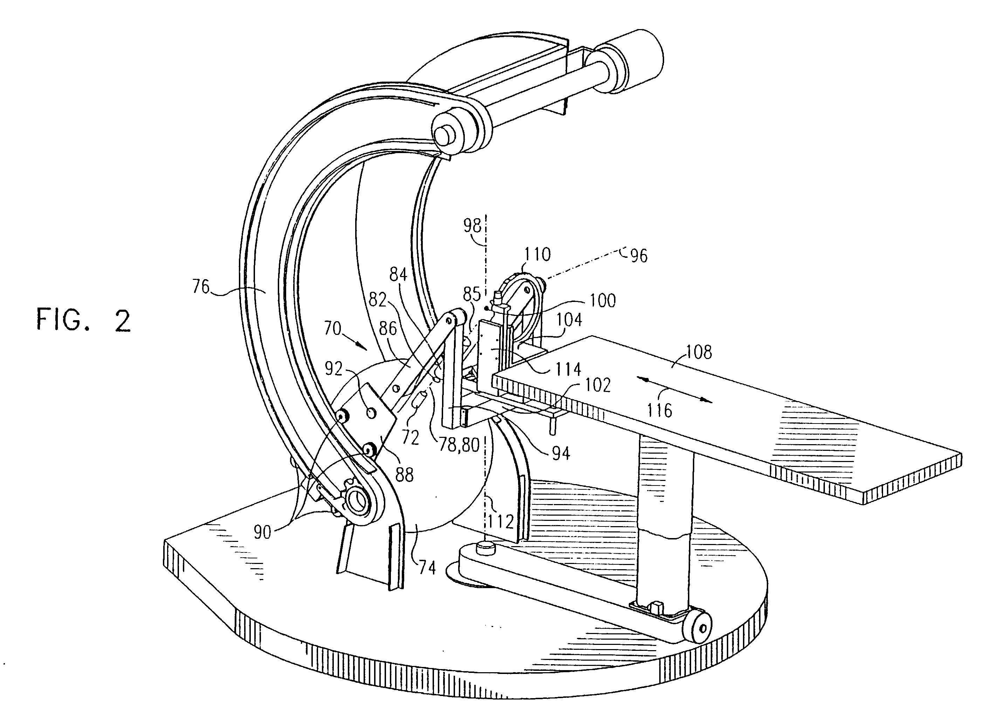

Reference is now made to Fig. 2 which illustrates a positioner 70 for stereotactic radiation treatment, constructed and operative in accordance with another preferred embodiment of the present invention.

Positioner 70 is particularly useful with a radiation system having a radiation source 72 housed in a radiation shield 74, typically generally spherical in shape, as seen in Fig. 2. Radiation shield 74 together with radiation source 72 are arranged to slide along a generally arcuate track 76. Track 76 may be circular, elliptic, parabolic or any other curvilinear shape. Radiation source 72 emits a radiation beam 78 along a radiation axis 80. Radiation beam 78 exits an aperture 82 formed in radiation shield 74.

Positioner 70 includes a source adapter arm 86 connected to radiation shield 74. Source adapter arm 86 is preferably attached to a flange member 88 provided with one or more roller elements 90 which slide along track 76, so that arm 86 moves together with radiation shield 74. The spatial position of arm 86 is thus determined by the position of radiation source 72. Radiation beam 78 is preferably collimated by a beam collimator 84 attached to arm 86 which defines a beam axis 85.

Preferably a source adapter arm positioning assembly 92 is provided for adjusting attachment of arm 86 with respect to radiation shield 74 and radiation source 72. Assembly 92 may be merely a plurality of adjustment screws or may include more elaborate adjustment equipment, such as a step motor or actuator Assembly 92 may adjust the position of arm 86 relative to radiation source 72 along and/or about any axis, such as beam axis 85 or a curvilinear axis corresponding to the shape of track 76, for example.

A frame adapter arm 94 is pivotably attached to source adapter arm 86 about a pivot axis 96. Arm 94 defines a frame axis 98 intersecting pivot axis 96. Pivot axis 96 and frame axis 98 are selected such that when arm 86 is fixed relative to radiation source 72, pivot axis 96 and frame axis 98 both intersect radiation axis 80. Preferably pivot axis 96 and frame axis 98 both intersect radiation axis 80 generally at the same location, and most preferably substantially at the same location, designated by reference numeral 100. Pivot axis 96 and frame axis 98 are preferably generally orthogonal.

Arm 94 includes a distal portion 102 which is preferably attached to a patient support frame 104 such that frame 104 is rotatable about frame axis 98 As seen in Fig. 2, frame axis 98 is preferably the azimuth axis and pivot axis 96 is preferably the elevation axis.

There is preferably provided a table 108 upon which a patient (not shown) may be supported. The head of the patient is affixed to a head ring 110 of frame 104 such that location 100 lies in a target of the patient which is to be irradiated. Table 108 is rotatable about a table axis 112, as is well known in the art. Table axis 112 ideally generally coincides with frame axis 98. It is a particular feature of the present invention that even if table axis 112 is misaligned with frame axis 98, source adapter arm 86 always positions location 100 in a desired orientation with respect to radiation source 72 and frame adapter arm 94 always positions location 100 in a desired orientation with respect to patient support frame 104, i.e., radiation beam 78 substantially intersects location 100 and the target no matter how radiation shield 74, radiation source 72 or table 108 has moved or sagged. Radiation source 72 thus "drags" the target of the patient with it no matter where it goes or how it sags, and accurate irradiation of the target is ensured.

A frame adapter arm positioning assembly 114, such as an X-Y-Z positioner, is preferably pivotably mounted to frame adapter arm 94 and attached to patient support frame 104. Assembly 114 adjusts mounting of patient support frame 104 to arm 94. Assembly l14 moves patient support frame 104 along three mutually orthogonal axes (typically corresponding to axes 96 and 98 and to a longitudinal table axis 116, for example), so that the target can be properly aligned with location 100

It will be appreciated by persons skilled in the art that the present invention is not limited by what has been particularly shown and described hereinabove. Rather the scope of the present invention includes both combinations and subcombinations of the features described hereinabove as well as modifications and variations thereof which would occur to a person of skill in the art upon reading the foregoing description and which are not in the prior art.

## Claims

1. A positioner useful in stereotactic radiation treatment of a patient by radiation from a radiation source from which a radiation beam is emitted along a beam axis, the positioner comprising:
a source adapter arm arranged to be attached to the radiation source such that the position of the source adapter arm is determined by the position of the radiation source; and
a frame adapter arm pivotably attached to said source adapter arm about a pivot axis, said frame adapter arm defining a frame axis intersecting said pivot axis,
said pivot axis and said frame axis being selected such that when said source adapter arm is attached to said radiation source, said pivot axis and said frame axis both intersect said beam axis.

2. A positioner according to claim 1 and wherein said pivot axis and said frame axis are selected such that when said source adapter arm is attached to said radiation source, said pivot axis and said frame axis both intersect said beam axis generally at the same location.

3. A positioner according to claim 1 and wherein said pivot axis and said frame axis are selected such that when said source adapter arm is attached to said radiation source, said pivot axis and said frame axis both intersect said beam axis substantially at the same location.

4. A positioner according to claim 1 and wherein said pivot axis and said frame axis are generally orthogonal.

5. A positioner according to any of the preceding claims and also comprising a source adapter arm positioning assembly which adjusts attachment of the source adapter arm to the radiation source.

6. A positioner according to any of claims 1-4 and also comprising a patient support frame pivotably mounted to said frame adapter arm.

7. A positioner according to any of claims 1-4 and also comprising a frame adapter arm positioning assembly which adjusts mounting of said patient support frame to said frame adapter arm.

8. A positioner according to any of claims 1-4 and also comprising a beam collimator mounted onto said source adapter arm for collimating said radiation beam.

9. A stereotactic radiation treatment system for treatment of a patient by radiation, the system comprising:
a radiation source from which a radiation beam is emitted along a beam axis; and
a positioner comprising:
a source adapter arm arranged to be attached to the radiation source such that the position of the source adapter arm is determined by the position ofthe radiation source; and
a frame adapter arm pivotably attached to said source adapter arm about a pivot axis, said frame adapter arm defining a frame axis intersecting said pivot axis,
said pivot axis and said frame axis being selected such that when said source adapter arm is attached to said radiation source, said pivot axis and said frame axis both intersect said beam axis.

10. A stereotactic radiation treatment system according to claim 9 and also comprising a patient support frame with respect to which a patient can be rigidly mounted such that a target area of the patient is located at an intersection of said pivot axis, said frame axis and said beam axis.

11. A stereotactic radiation treatment system according to claim 9 or claim 10 and wherein said pivot axis and said frame axis both intersect said beam axis generally at the same location.

12. A stereotactic radiation treatment system according to claim 9 or claim 10 and wherein said pivot axis and said frame axis both intersect said beam axis substantially at the same location.

13. A stereotactic radiation treatment system according to claim 9 or claim 10 and wherein said pivot axis and said frame axis are generally orthogonal.

14. A stereotactic radiation treatment system according to claim 9 or claim 10 and also comprising a source adapter arm positioning assembly which adjusts attachment of the source adapter arm to the radiation source.

15. A stereotactic radiation treatment system according to claim 9 or claim 10 and also comprising a frame adapter arm positioning assembly which adjusts mounting of said patient support frame to said frame adapter arm.

16. A stereotactic radiation treatment system according to claim 9 or claim 10 and also comprising a beam collimator mounted onto said source adapter arm for collimating said radiation beam.

17. A stereotactic radiation treatment system according to claim 9 or claim 10 and wherein said source adapter arm is suspended from said radiation source.

18. A stereotactic radiation treatment system according to claim 9 or claim 10 and wherein said source adapter arm is at least partially suspended from said radiation source.

19. A stereotactic radiation treatment system according to claim 9 or claim 10 and wherein said source adapter arm and said radiation source are slidingly attached to a generally arcuate track.

20. A stereotactic radiation treatment system according to claim 9 or claim 10 and comprising a support element for supporting at least one of said source adapter arm and said frame adapter arm.

21. A stereotactic radiation treatment system according to claim 9 or claim 10 and comprising a gantry in which is housed said radiation source, said gantry having a gantry arm which rotates about a gantry arm axis, wherein said pivot axis is offset from said gantry axis.
